# EUROPEAN PATENT APPLICATION

(11) **EP 4 372 371 A1**
(43) Date of publication of application: **22.05.2024**
(21) Application number: 22842205.1
(22) Date of filing: 15.07.2022
(51) Int. Cl.: G01N 27/30, G01N 27/26, G01N 27/27, G01N 27/416

(54) **SYSTEM FOR INFERRING DYNAMIC STATE OF SYSTEM TO BE MEASURED USING REDOX POTENTIAL**

(30) Priority: 15.07.2021 JP 2021117114
(71) Applicant: Chitose Laboratory Corp., Kawasaki-shi, Kanagawa 216-0041 (JP)
(72) Inventor: SHONO, Nobuaki, Kawasaki-shi, Kanagawa 216-0041 (JP); OKADA, Katsunori, Kawasaki-shi, Kanagawa 216-0041 (JP); TAKASHIMA, Masatoshi, Kawasaki-shi, Kanagawa 216-0041 (JP); KASAHARA, Ken, Kawasaki-shi, Kanagawa 216-0041 (JP); FUJITA, Tomohiro, Kawasaki-shi, Kanagawa 216-0041 (JP)
(74) Representative: van Dam, Vincent
(86) International application number: PCT/JP2022/027895
(87) International publication number: WO 2023/286864

(57) **Abstract**

[Problem] To provide a system for inferring the dynamic state of a system to be measured, said system for inferring able to acquire data that are stable and correlated with respect to changes in the system to be measured through the ascertainment and management of the operating state of electrodes of the system for inferring the dynamic state of a system to be measured. [Solution] A system 1 for inferring the dynamic state of a system to be measured includes a reference electrode 3, a multi-electrode probe 11 that includes a plurality of working electrodes 5, 7, 9 that are mutually insulated, and a potential measuring/voltage applying unit 13 that measure the redox potential of the reference electrode 3 and one or more working electrodes among the plurality of working electrodes 5, 7, 9, wherein: the multi-electrode probe 11 comprises a tip section 17 that contacts a measurement subject; and the plurality of working electrodes 5, 7, 9 are exposed at least at the tip section 17.

## Description

### TECHNICAL FIELD

The invention relates to a system for inference of dynamics of a system to be measured by using redox potentials.

### BACKGROUND ART

Patent Document 1 describes a system for inference of dynamics of a system to be measured by using redox potentials.

In the system for inference of dynamics of a system to be measured (Patent Document 1), when data that are stable and correlated to changes in the system to be measured are acquired, it has been difficult to constantly maintain the surface condition and sterilization condition of a plurality of electrodes in good condition. In addition, there has been a problem in ensuring the reliability of measurement data. Furthermore, there has been a problem in a version management method and scalability of electrode types and the like.

Patent Document 1: JP-A-2021-043097

### SUMMARY OF INVENTION

### TECHNICAL PROBLEM

An object of one aspect of the invention described herein is to provide a system for inference of dynamics of a system to be measured capable of acquiring data that are stable and correlated to changes in the system to be measured by grasping and managing operating states of electrodes in the system for inference of dynamics of the system to be measured.

### SOLUTIONS TO PROBLEMS

One aspect of the invention described herein is a system for inference of dynamics of a system to be measured including a reference electrode, a multi-electrode probe, and a potential measuring and voltage applying unit, and has the following aspects. The multi-electrode probe includes a plurality of working electrodes in a state of being insulated from one another. The potential measuring and voltage applying unit measure a redox potential related to the reference electrode and any one or at least two working electrodes included in the plurality of working electrodes. The multi-electrode probe has a distal end portion in contact with a measurement target. The plurality of working electrodes are exposed at least at the distal end portion.

In a preferable aspect of the invention described herein, the distal end portion has a flat shape.

In a preferable aspect of the invention described herein, the distal end portion has a shape inclined with respect to a longitudinal direction of the multi-electrode probe.

In a preferable aspect of the invention described herein, the system for inference of dynamics includes a connection unit and has the following aspect. The potential measuring and voltage applying unit are connected to the multi-electrode probe via the connection unit.

In a preferable aspect of the invention described herein, the multi-electrode probe further includes a ground electrode further insulated from the plurality of working electrodes.

In a preferable aspect of the invention described herein, each of the plurality of working electrodes has a diameter of 0.5 mm or more and 3 cm or less.

In a preferable aspect of the invention described herein, the respective plurality of working electrodes have different ionization tendencies.

In a preferable aspect of the invention described herein, the system for inference of dynamics includes a status confirmation unit and has the following aspect. The status confirmation unit performs status confirmation of the system for inference of dynamics of a system to be measured based on the redox potential measured by the potential measuring and voltage applying unit.

In a preferable aspect of the invention described herein, the status confirmation unit of the system for inference of dynamics has the following aspects. The potential measuring and voltage applying unit apply a plurality of types of voltages to at least one of the plurality of working electrodes. Based on the redox potential when the potential measuring and voltage applying unit apply a plurality of types of voltages to at least one of the plurality of working electrodes, the status confirmation unit performs status confirmation of the system for inference of dynamics of a system to be measured.

In a preferable aspect of the invention described herein, the system for inference of dynamics includes an identification information recording unit, an identification information readout unit, and working electrode analyzing unit, and has the following aspects. The identification information readout unit reads out the identification information of the multi-electrode probe from the identification information recording unit. The working electrode analyzing unit analyzes the plurality of working electrodes included in the multi-electrode probe based on the identification information of the multi-electrode probe read out by the identification information readout unit.

In a preferable aspect of the invention described herein, the plurality of working electrodes include two or more working electrodes having an identical cross-sectional shape and made of identical material. By comparing potentials derived from the plurality of electrodes having an identical material, an accurate potential can be obtained. For example, when the potentials obtained by the respective electrodes are the same, it can be said that the accurate potential has been obtained.

The next invention includes a plurality of multi-electrode probes. The system for inference of dynamics of a system to be measured includes a reference electrode, a plurality of multi-electrode probes, and a potential measuring and voltage applying unit that measure a redox potential related to the reference electrode and any one or at least two working electrodes included in the plurality of working electrodes. Each of the plurality of multi-electrode probes includes a plurality of working electrodes in a state of being insulated from one another and a distal end portion in contact with a measurement target, and the plurality of working electrodes are exposed at least at the distal end portion.

### ADVANTAGEOUS EFFECTS OF INVENTION

One aspect of the invention described herein can provide a system for inference of dynamics of a system to be measured.

### BRIEF DESCRIPTION OF DRAWINGS

Fig. 1 is a diagram illustrating a whole image of a system configuration for inference of dynamics of the present invention.
Fig. 2 is a diagram illustrating an exemplary configuration of a control system in the system configuration for inference of dynamics of the present invention.
Fig. 3 is a diagram illustrating an aspect of constituting equipment of the system for inference of dynamics of the present invention.
Fig. 4-1 is a conceptual diagram illustrating an exemplary aspect of a multi-electrode probe.
Fig. 4-2 illustrates exemplary shapes of the multi-electrode probe in the present invention.
Fig. 5 is a diagram illustrating an exemplary configuration of an identification information readout unit and an identification information recording unit.
Fig. 6 is a diagram partially illustrating an exemplary aspect of a culture measurement system and a control system of the present invention.
Fig. 7 is a diagram for describing an exemplary aspect of constituting equipment of the system for inference of dynamics of the present invention.
Fig. 8-1(a) is a diagram illustrating a relationship between an electrode potential generated when a metal electrode is dipped in an aqueous solution (water) and a pH value.
Fig. 8-2(b) and Fig. 8-2(c) are diagrams illustrating the relationship between the electrode potential generated when a metal electrode is dipped in an aqueous solution (water) and the pH value.
Fig. 9 is a diagram illustrating a specific example related to the relationship between the electrode potential and pH.
Fig. 10 is a diagram illustrating an exemplary processing flow of the present invention.
Fig. 11 is a diagram illustrating a part of the processing flow of Fig. 10 in detail.
Fig. 12(a) is a conceptual diagram illustrating an example of an electrode probe having a plurality of electrodes made of an identical material. Fig. 12(b) is a graph substituting a drawing illustrating potentials measured using the electrode probe illustrated in Fig. 12(a) .
Fig. 13 is a conceptual diagram of a system including a plurality of multi-electrode probes.

### DESCRIPTION OF EMBODIMENTS

Embodiments of the present invention will be described below with reference to the drawings. The present invention is not limited to the embodiments described below, and includes modifications of the following embodiments as appropriate within a scope obvious to those skilled in the art.

### <Outline of Invention>

In a system for inference of dynamics of a system to be measured by using redox potentials (Patent Document 1), at least three different electrodes (different in points, such as material, surface roughness, and composition) are used, and what has been focusing on is that potential differences obtained from these electrodes relative to a reference electrode depend on a state of target dynamics, and a wide variety of information is superimposed. Therefore, it has been indicated that, utilizing these pieces of data as multivariate data for machine learning and the like enables inference of dynamics, which can be applicable in various fields of biochemical phenomena.

Examples of the dynamics of a system to be measured include, for example, the survival conditions, activity conditions, dispersion conditions, proliferation conditions, fermentation status, amount of predetermined metabolites, amount of decomposition products, conditions suitable for a certain purpose, and conditions unsuitable for a certain purpose of the organisms living in a system to be measured.

For example, a system 1 for inference of dynamics stores potential information during normal fermentation of a certain substance as data, on which machine learning is performed. Then, when the potential information of a new system is different from any of the machine-learned potential information patterns, it is expected that the conditions for fermentation are not normal and the quality of the substance may change. Using a plurality of pieces of potential information as data in this way, for example, makes it possible to keep the quality of beverages such as beer constant.

On the other hand, in the present invention, various ingenuities are performed from a viewpoint of how it is possible to implement maintenance control and quality control over three or more different electrodes in an easy-to-use manner, realizing improvement in performance and efficiency of the system for inference of dynamics of a system to be measured by using redox potentials (Patent Document 1).

### <Description of Each Element>

### (System for Inference of Dynamics)

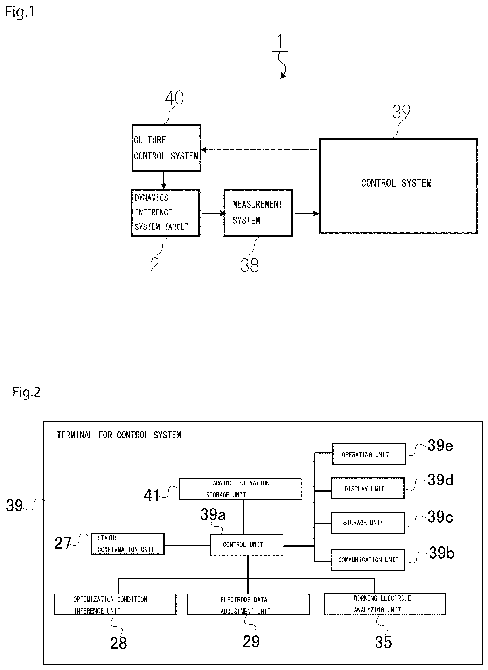

A system 1 for inference of dynamics of the present invention includes a reference electrode 3, a multi-electrode probe 11, a potential measuring and voltage applying unit 13, and a potential information and dynamics information storage unit 15. The multi-electrode probe 11 includes a plurality of working electrodes 5, 7 and 9 in a state of being insulated from one another. As illustrated in Fig. 1, the system 1 for inference of dynamics of the present invention may include a dynamics inference system target (culture tank) 2, a measurement system 38, a control system 39, and a culture control system 40.

As illustrated in Fig. 2, the control system 39 may have a control unit 39a, a communication unit 39b, a storage unit 39c, a display unit 39d, an operating unit 39e, and a learning estimation storage unit 41.

The control unit 39a is means for executing controls of respective units, such as transmission and reception of information, storage of information in the storage unit, and control of display contents on the display unit.

The communication unit 39b is configured from a communication interface and has a function to transmit and receive information between respective systems of the system 1 for inference of dynamics.

The storage unit 39c has a function to store various information for actions of the system 1 for inference of dynamics. For example, the storage unit 39c stores programs for the actions of the system 1 for inference of dynamics and stores process results of the respective systems.

The display unit 39d is an element for displaying visual information. The display unit 39d may be, for example, a display device of various kinds.

The operating unit 39e is an element for accepting input of operation information from a user of each terminal described above. The operation information input via the operating unit is transferred to the control unit to be made available for controlling each terminal. For the operating unit 39e, various kinds of keyboards, mice, and touch panels used for known information processing terminals can be employed. Further, a touch panel constituting the operating unit may constitute a touch panel display together with a display constituting the display unit.

Note that the measurement system 38 and the culture control system 40 may also have control units, communication units, storage units, display units, and operating units that have similar functions as those described above.

Other than the example described above, a configuration in which a server in the cloud is utilized as the control system 39 to allow a more complex computation can be employed. By transferring measurement data to a server in the cloud via the Internet by a communication system and utilizing the computing power of the server having powerful performance in the cloud, a more complex and more accurate learning estimation system can be operated.

The measurement system 38 may include a potential measurement instrument, other measurement instruments, and the like. Signal data output from the measurement system 38 are transmitted to the control system 39. Then, the data are used as multivariate data for machine learning and the like in the learning estimation system and storage.

The result is transmitted to the culture control system 40 to control the dynamics inference system target (culture tank), thereby executing more optimal culture control.

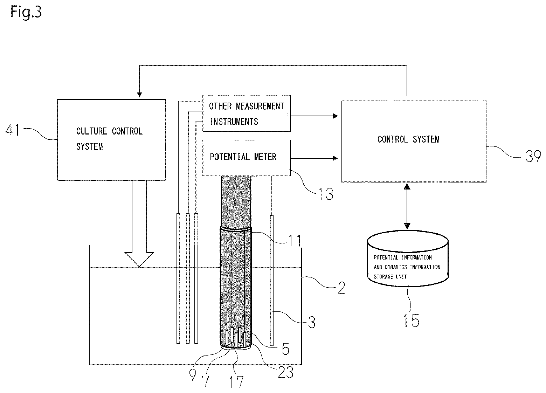

As illustrated in Fig. 3, the system 1 for inference of dynamics of a system to be measured has the reference electrode 3 and first working electrodes 5, 7, and 9. Furthermore, the system 1 for inference of dynamics of a system to be measured preferably has the potential information and dynamics information storage unit 15.

The system 1 may further have an optimization condition inference unit 28. The optimization condition inference unit 28 can read out the data recorded in the learning estimation storage unit 41 to perform various analyses, thereby inferring optimization conditions.

### (Working Electrode, Reference Electrode, Ground Electrode)

The working electrodes 5, 7, and 9 are electrodes that work on a measurement target to measure the redox potential between each working electrode and the reference electrode 3. At least three working electrodes are prepared. The system 1 for inference of dynamics of a system to be measured may include three or more (for example, four, five, six, or seven) working electrodes. Each of the plurality of working electrodes is preferably insulated. In addition, it is preferable that a potential meter is provided between each working electrode and the reference electrode 3 to measure the potential difference independently.

The working electrodes 5, 7, and 9 may be any electrode as long as it is conductive and can come into contact with an object (typically containing a liquid). Each working electrode is different at least in material or surface treatment from the other electrodes. Examples of materials of the working electrodes include platinum, gold, carbon, carbon allotropes (glassy carbon, diamond, graphene, carbon nanotubes, fullerenes), and alloys. The different material referred to herein includes one with a different composition ratio. The different surface treatment referred to herein includes one with a different electrode surface roughness, different atomic arrangement on the electrode surface, and/or different shape. The shape of each working electrode may be adjusted as appropriate according to the application.

The above plurality of working electrodes are preferably integrated into one multi-electrode probe 11 in a state where each working electrode is kept insulated from one another.

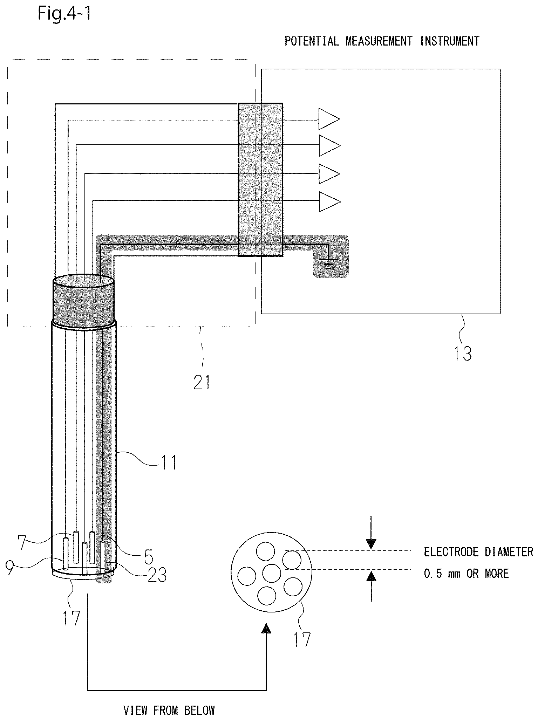

Fig. 4-1 is a conceptual diagram illustrating an exemplary aspect of the multi-electrode probe 11. As illustrated in Fig. 4-1, the shape of the working electrodes may be, for example, a column shape. In this case, the above working electrodes preferably have a diameter of 0.5 mm or more and 30 mm or less. In addition, it is preferable that the multi-electrode probe 11 further includes a ground electrode 23 insulated from the above working electrodes.

Generally, when the diameter of the working electrodes of the multi-electrode probe 11 as described above is set to be less than 0.5 mm, the area of the multi-electrode probe that comes into contact with a solution decreases, and the sensitivity to changes in potential increases. Although high sensitivity to changes in potential enables following an abrupt change in potential, it causes a state of being vulnerable to noise.

As a countermeasure against noise, the number of samplings can be increased for avoidance. However, when the number of samplings becomes too large, acquired data becomes too large, increasing the impact of data transfer cost and storage cost.

Meanwhile, from the relationship between measurement frequency and sensitivity, when the diameter of the working electrodes of the multi-electrode probe 11 is 0.5 mm or more, stable data can be acquired.

The multi-electrode probe 11 may have a distal end portion 17 having a flat shape. The plurality of working electrodes 5, 7, and 9 are preferably exposed at least at the distal end portion 17.

The respective plurality of working electrodes 5, 7, and 9 preferably have different ionization tendencies.

The reference electrode 3 is an electrode that works on a measurement target to measure the redox potentials between the reference electrode 3 and the working electrodes 5, 7, and 9.

### (Multi-electrode Probe)

While a plurality of electrodes are installed for redox potential measurement in the conventional invention, these are integrated into one electrode. Specifically speaking, the plurality of electrodes are integrated into a form of one electrode in a state where the respective electrodes are kept insulated. Then, a part of the multi-electrode probe 11 that comes into contact with a system to be measured may be molded into a planar surface. In Fig. 4-1, the multi-electrode probe 11 is illustrated in a state where a plurality of electrodes are integrated into one multi-electrode probe 11. In the multi-electrode probe 11, various information is superimposed compared with the conventional electrodes for redox potential measurement. By integrating the plurality of electrodes into one and arranging the part where the electrodes (all the plurality of electrodes) come into contact with a measurement target on one planar surface, the area of the electrodes that can come into contact with a solution can be unified. In addition, by performing cleaning, polishing, and the like, the state of the surface can be always kept constant and controlled.

Moreover, by shaping the bottom surfaces of the electrodes to be inclined surfaces, air bubbles during culture remaining on the bottom surfaces can be avoided, and stable data can be constantly acquired.

The multi-electrode probe 11 includes the above plurality of working electrodes in a state where those working electrodes are insulated from one another. Furthermore, the multi-electrode probe 11 has the distal end portion 17 that comes into contact with a measurement target (such as a culture solution). The multi-electrode probe 11 may have the distal end portion 17 having a flat shape. The above plurality of working electrodes are preferably exposed at least at the distal end portion 17.

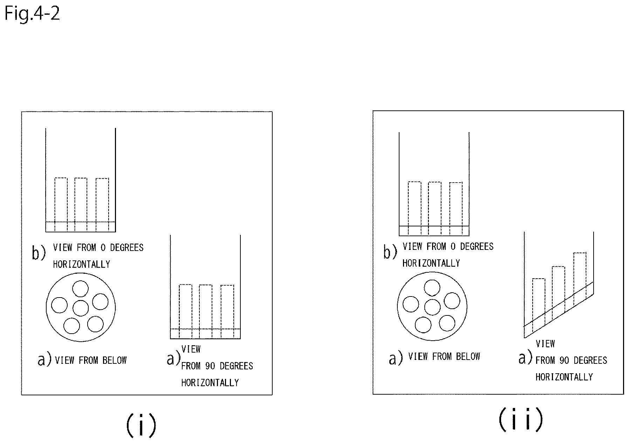

The distal end portion 17 having a flat shape may be configured such that the electrode shape is perpendicular to the electrode bottom surface as illustrated in Fig. 4-2 (i), and may have a shape of being cut at an angle as illustrated in Fig. 4-2(ii), which is a shape inclined with respect to a longitudinal direction of the multi-electrode probe 11.

Furthermore, the multi-electrode probe 11 preferably includes an identification information recording unit 31 that stores identification information of the multi-electrode probe 11. The system 1 for inference of dynamics has an identification information readout unit 33 that reads out the identification information of the multi-electrode probe from the identification information recording unit 31 and a working electrode analyzing unit 35.

Since various information is superimposed to be out compared with the conventional redox potentials, the multi-electrode probe 11 is referred to as a "convolutional electrode probe."

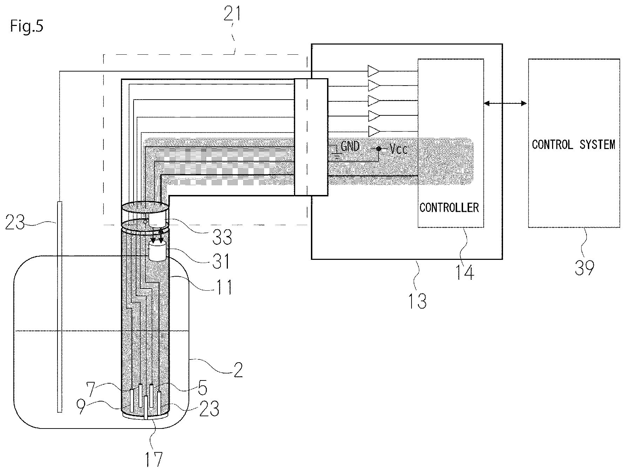

Fig. 5 is a diagram illustrating an exemplary configuration of the identification information reading unit 33 and the identification information recording unit 31. As illustrated in Fig. 5, the identification information recording unit 31 that holds information and the like on the version, serial number, and electrode type (electrode ID and profile information) of the multi-electrode probe 11 is preferably provided in the multi-electrode probe 11 (convolutional electrode probe). In addition, a mechanism that performs power supply to a connector unit 21 connecting the multi-electrode probe 11 to the potential measuring and voltage applying unit 13 and executes data reading processing and registration processing with respect to the identification information recording unit 31 may be provided.

The information of the multi-electrode probe 11 (convolutional electrode probe) may be stored in the identification information recording unit 31 in advance, and the information may be read out before measurement start, transmitted to the control system 39, and recorded in Header information and the like of measurement data.

The multi-electrode probe (convolutional electrode probe) may be, for example, one in which five electrodes (Pt: platinum, Au: gold, Ag: argentum, Cu: copper, SUS: stainless steel) are used.

The above electrodes may have a structure configured by cutting each out into about 1 cm to 2 cm, connecting a lead wire to each electrode, and putting the electrodes into one pipe to be packed with resin. This enables complete insulation between the electrodes and avoidance of liquid getting through from a contact portion with an aqueous solution. In this example, the five electrodes constitute one surface in an aspect in which the bottom surface of the multi-electrode probe 11 is cut out at an angle.

### (Identification Information Recording Unit)

The multi-electrode probe 11 preferably has the identification information recording unit 31 that stores the identification information of the multi-electrode probe 11. For example, the identification information recording unit 31 may have a multi-electrode probe table and a multi-electrode probe internal electrode table defined by a relational database as below. For example, column items of the multi-electrode probe table may include multi-electrode probe ID and multi-electrode probe version. In this case, the multi-electrode probe ID may be set as a main key of the multi-electrode probe table. In addition, column items of the electrode table may be, for example, multi-electrode probe ID, electrode ID, electrode type, and computation method, correction method, and abnormal value data range of electrodes. In this case, the combination of the multi-electrode probe ID and electrode ID may be set as a main key.

### (Identification Information Readout Unit)

The system 1 for inference of dynamics of a system to be measured preferably has the identification information readout unit 33 that reads out the identification information of the multi-electrode probe 11 from the identification information recording unit 31. This element may be a known mechanism as long as it can read out the information from the identification information recording unit 31.

### (Potential Measuring and Voltage Applying Unit, and Electrode State Grasping Processing)

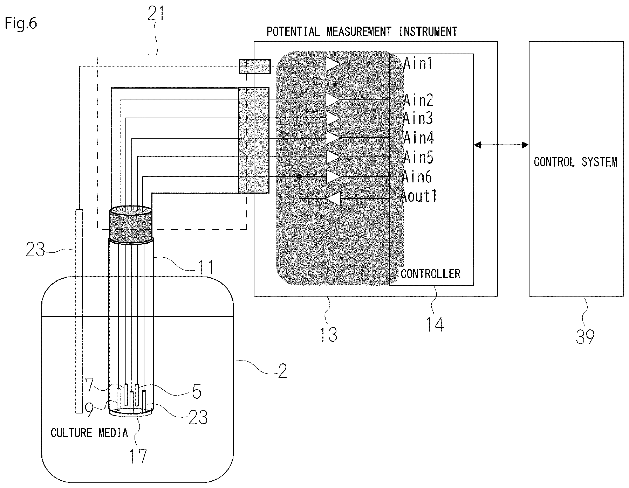

Fig. 6 is a diagram partially illustrating an exemplary aspect of a culture measurement system and a control system of the present invention. The system 1 for inference of dynamics of a system to be measured preferably includes the potential measuring and voltage applying unit 13 that measure the redox potential related to any one or at least two working electrodes included in the plurality of working electrodes 5, 7, and 9. The potential measuring and voltage applying unit 13 may be an element included in a known potential measurement instrument.

In addition, the system 1 for inference of dynamics of a system to be measured may be in an aspect in which a plurality of types of voltages are applied to at least one of the plurality of working electrodes 5, 7, and 9.

Fig. 6 is a diagram illustrating a specific example of the potential measuring and voltage applying unit 13. In the example of Fig. 6, the potential measurement instrument has seven Op-Amps. In this case, one of them may function as a reference electrode. In addition, five Op-Amps different from the above reference electrode are connected to the (multi-electrode probe) convolutional electrode probe and perform impedance conversion of converting a signal with a large output impedance into a signal with a small output impedance.

These signals may be put into a controller 14, and subjected to A/D conversion and signal processing internally. The remaining one Op-Amp other than the above six Op-Amps may be used for driving signal output of Aout1 described below.

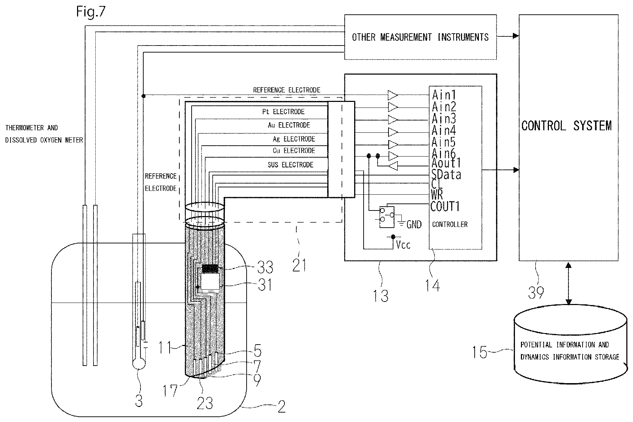

Fig. 7 is a diagram illustrating an exemplary aspect of the culture measurement system and the control system of the present invention, which is a diagram for describing electrode state grasping processing. For example, the potential measuring and voltage applying unit 13 may have a relay switch as illustrated in Fig. 7. In this case, with the relay switch, the state of the ground electrode 23 (such as a SUS electrode) of the multi-electrode probe 11 can be switched between a state of being connected to ground and an open state (a floating state in terms of potential). When a normal dynamics measurement is performed, the ground electrode 23 may constantly be connected to ground. On the other hand, when the electrode state grasping processing to confirm the state of the multi-electrode probe 11 is executed, the ground electrode 23 is disconnected from ground and enters the open state.

Next, the electrode state grasping processing of the multi-electrode probe 11 (convolutional electrode probe) will be described. This processing is processing for checking the operating states of the plurality of electrodes included in the multi-electrode probe 11. A specific example of processing will be described below with reference to Fig. 7. First, based on inputs and the like to an operating unit by an operator, the control unit of a measurement target system 38 controls COUT1 of the controller 14 of the potential measuring and voltage applying unit 13 to open the relay switch of the potential measuring and voltage applying unit 13 (the above open state). Next, 0 V is output at Aout1 to measure the potentials from Ain1 to Ain6. Next, Aout1 is switched to 2.5 V to measure the potentials from Ain1 to Ain6. A status confirmation unit 27 examines the potential differences of the respective electrodes (Ain1 to Ain6) at 0 V and 2.5 V to confirm whether or not the connection of the electrodes is normal. In addition, natural potentials at 0 V are observed to confirm that the states of the respective electrodes are normal. Based on the inputs to the operating unit by the operator, the control unit of the measurement system 38 controls COUT1 and closes the relay switch, causing the ground electrode 23 to connect to ground and the measurement target system to return to the normal dynamics measurement.

The electrode state grasping processing is executed for grasping the states of the respective electrodes of the multi-electrode probe 11 (convolutional electrode probe) before culture start. However, after the actual culture starts, the states of the electrodes are appropriately grasped periodically or according to the electrode state and provide feedback to electrode data adjustment processing (edge processing), thereby allowing the electrode data adjustment processing (edge processing) described later to be changed in real-time.

### (Status Confirmation Unit)

The system 1 for inference of dynamics of a system to be measured preferably includes the status confirmation unit 27 that performs status confirmation of the system 1 for inference of dynamics of a system to be measured based on the redox potential when the potential measuring and voltage applying unit 13 apply a plurality of types of voltages to at least one of the plurality of working electrodes 5, 7, and 9. The status confirmation unit 27 may confirm that the states of the respective electrodes are normal based on the potential information of the respective electrodes in the electrode state grasping processing described above.

### (Potential Information and Dynamics Information Storage Unit)

Fig. 7 is a diagram for describing an exemplary aspect of constituting equipment of the system for inference of dynamics of the present invention. The system 1 for inference of dynamics of a system to be measured preferably includes the potential information and dynamics information storage unit 15 that stores one or at least two redox potentials measured by the potential measuring and voltage applying unit 13. The potential information and dynamics information storage unit 15 may be included in the measurement system 38. The potential information and dynamics information storage unit 15 may have a potential information table defined by, for example, a relational database. Column items of the potential information table may be multi-electrode probe ID, electrode ID, measurement ID, electrode potential, and electrode operating information (information on normal or not). In the case of this example, the combination of the multi-electrode probe ID, electrode ID, and measurement ID may be set as a main key of this table.

In addition, the potential information and dynamics information storage unit 15 may have a dynamics information table. Column items of the dynamics information table may be, for example, measurement ID, temperature, humidity, atmospheric pressure, turbidity and acidity of culture solution, concentration of a target substance, and presence/absence of a target substance. In this case, the measurement ID may be set as a main key of this table.

### (Connection Unit)

The system 1 for inference of dynamics of a system to be measured preferably includes a connection unit 21 that removably connects the multi-electrode probe 11. The potential measuring and voltage applying unit 13 are connected to the multi-electrode probe 11 via the connection unit 21. In addition, the multi-electrode probe 11 is connected to the potential measuring and voltage applying unit 13 via the connection unit 21. The connection unit 21 may be a known mechanism as long as the above functions are fulfilled. A specific example of connecting the connection unit 21 to the potential measuring and voltage applying unit 13 may be a cable and the like.

### (Working Electrode Analyzing Unit and Electrode Data Adjustment Unit)

The system 1 for inference of dynamics of a system to be measured preferably has the working electrode analyzing unit 35 for analyzing the plurality of working electrodes included in the multi-electrode probe 11 based on the identification information of the multi-electrode probe 11 read out by the identification information readout unit 33.

The working electrode analyzing unit 35 may be an element for specifying abnormal electrodes based on the data acquired by the measurement system and executing computation processing for each electrode. Furthermore, the working electrode analyzing unit 35 may have a function to specify a correction method of preset electrode data. In addition, the working electrode analyzing unit 35 may have a function for specifying a data range to judge that electrode data are abnormal.

The system 1 for inference of dynamics of a system to be measured may have an electrode data adjustment unit 29. The electrode data adjustment unit 29 may perform exclusion (data filtering) or adjustment (correction or calibration) of acquired electrode data by preset parameters and the like. In addition, the electrode data adjustment unit 29 may specify electrodes that should be judged as abnormal based on the preset abnormal data range of the electrode data to judge abnormal electrodes and the operating states of the electrodes grasped by the status confirmation unit 27.

Specific processing for them will be described in an overall processing flow described below.

### (About Selection of Electrode)

Combinations of the above working electrodes will be described below.

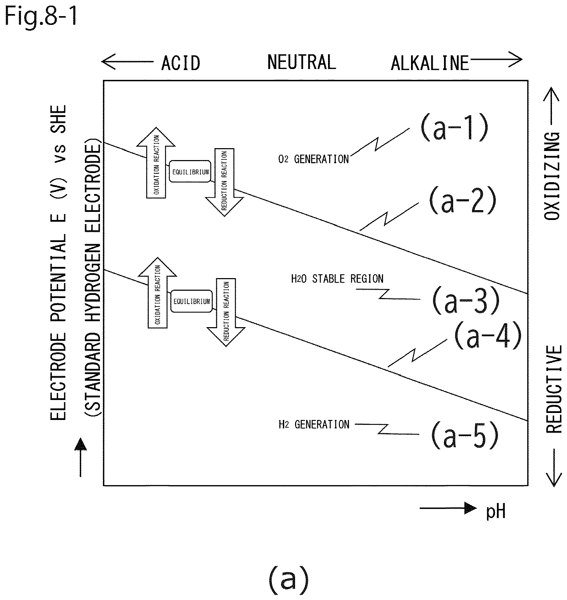

Fig. 8-1(a) illustrates an electrode potential generated when a metal electrode is dipped in an aqueous solution (water) using a "potential-pH diagram." The horizontal axis indicates the pH value, and the vertical axis indicates the electrode potential when a standard hydrogen electrode is used as a reference. As going upward on the vertical axis, the aqueous solution has stronger oxidizing properties, and the electrode potential becomes higher. As going downward on the vertical axis, the aqueous solution is reductive, and the electrode potential becomes lower.

Under conditions of the potential and the pH value on a straight line 2 in Fig. 8-1(a), a redox reaction expressed by the formula of "O₂ + 4H(+) + 4e(-) 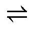 2H₂O" is in equilibrium.

In Fig. 8-1(a), a region on an upper side with respect to the straight line 2 is a region corresponding to the conditions in a case where the reaction of "O₂ + 4H (+) + 4e(-) ← 2H₂O" occurs more often than the reaction of "O₂ + 4H(+) + 4e(-) → 2H₂O."

In Fig. 8-1(a), a region on a lower side with respect to the straight line 2 and on the upper side with respect to a straight line 4 is a region corresponding to the conditions in a case where the reaction of "O₂ + 4H (+) + 4e(-) → 2H₂O" occurs more often than the reaction of "O₂ + 4H(+) + 4e(-) , 2H₂O."

Under conditions of the potential and the pH value on a straight line (a-4) in Fig. 8-1(a), a redox reaction expressed by the formula of "2H₂O + 2e (-) # H₂ + 2OH (-)" is in equilibrium.

In Fig. 8-1(a), a region on the lower side with respect to a straight line (a-2) and on the upper side with respect to the straight line (a-4) is a region corresponding to the conditions in a case where the reaction of "2H₂O + 2e(-) ← H₂ + 2OH(-)" occurs more often than the reaction of "2H₂O + 2e(-) → H₂ + 2OH(-). "

In Fig. 8-1(a), a region on the lower side with respect to the straight line (a-4) is a region corresponding to the conditions in a case where the reaction of "O₂ + 4H (+) + 4e(-) , 2H₂O" occurs more often than the reaction of "O₂ + 4H(+) + 4e(-) → 2H₂O."

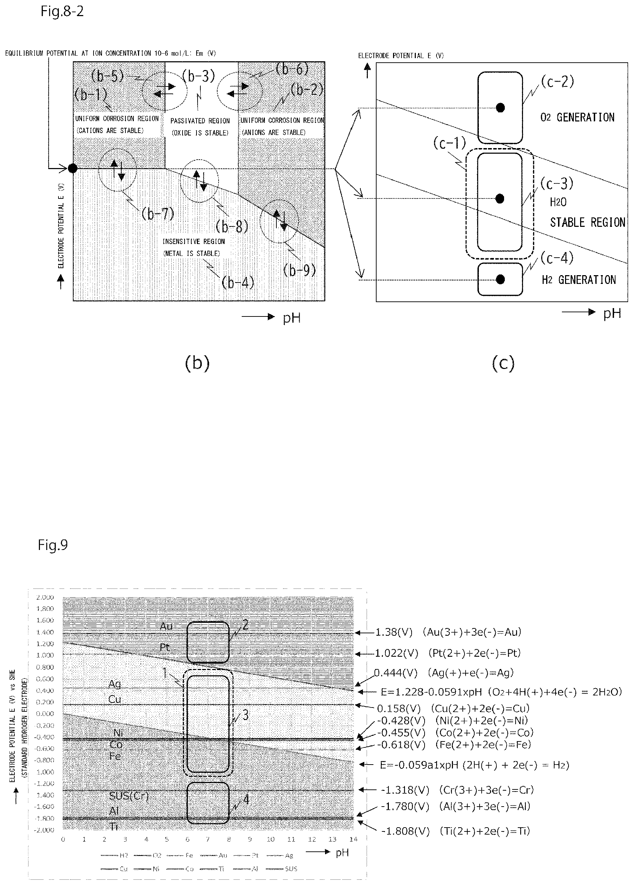

Fig. 8-2(b) illustrates an electrode potential generated when a metal electrode X is dipped in an aqueous solution (water) using a "potential-pH diagram."

The horizontal axis indicates the pH value, and the vertical axis indicates the electrode potential when a standard hydrogen electrode is used as a reference.

A polygonal line in Fig. 8-2(b) indicates a relationship between the potential and the pH value when a metal X is dipped in a certain aqueous solution. The metal X has an equilibrium potential of Em (V) when an ion concentration is 10⁻⁶ mol/L.

A lower region (region (b-4)) with respect to the polygonal line is a region indicating a case where an equilibrium potential of the aqueous solution is lower than an equilibrium potential of the redox potential of the electrode itself. On the other hand, upper regions ((b-1), (b-2), and (b-3)) with respect to the polygonal line are regions indicating a case where the equilibrium potential of the aqueous solution is higher than the equilibrium potential of the redox potential of the electrode itself. In a state of the region (b-4), the above metal X is stable and in an insensitive region state.

Conversely, in a state where the equilibrium potential of the aqueous solution is higher than the equilibrium potential of the redox potential of the electrode itself, it depends on the pH value (acidity, alkalinity) of the aqueous solution. In an acid region with a low pH value, the metal is oxidized, resulting in a corrosion region where cations are stable. This corresponds to the region (b-1) in Fig. 8-2.

When the equilibrium potential of the aqueous solution is higher than the equilibrium potential of the redox potential of the electrode itself and the pH is in the middle, an oxide is formed on the metal surface, and it becomes stable, therefore resulting in a passivated region.

This corresponds to the region (b-3) in Fig. 8-2 (b) . When the equilibrium potential of the aqueous solution is higher than the equilibrium potential of the redox potential of the electrode itself and the pH value further becomes higher, alkali corrosion occurs, therefore resulting in a region where anions are stable. This corresponds to the region (b-2) in Fig. 8-2(b).

In addition, at interfaces (b-5), (b-6), (b-7), (b-8), and (b-9) of these four regions, two regions are in equilibrium. Positions (relationship between potential and pH value) of the interfaces vary according to metals.

Next, Fig. 8-2(c) will be described. An electrode in a region (c-2) in Fig. 8-2(c) is a metal in the insensitive region where the metal is stable. Since the electrode in this region does not react as a metal, the equilibrium potential that the aqueous solution itself has appears as the electrode potential. For an electrode in a region (c-3) in Fig. 8-2(c), the states of (c-1), (c-2), (c-3), and (c-4) illustrated in Fig. 8-2(b) vary according to the state of the aqueous solution as a dynamics inference system target. Therefore, various information is superimposed and can be extracted as the electrode potential. An electrode in a region (c-4) in Fig. 8-2(c) has an oxide formed on the metal surface and is in the passivated region where the oxide is stable. For the electrode, the equilibrium potential between the redox potential of the oxide and the redox potential of the aqueous solution appears as the electrode potential. Thus, by employing materials having significantly different features as the convolutional electrode probe (multi-electrode probe 11), data become effective when utilized as multivariate data for machine learning and the like.

When the equilibrium potential of the aqueous solution as a dynamics inference system target varies within a region (1) in Fig. 8-2(c), a group of electrodes in the potential region (c-2) where the equilibrium potential (Em (V)) at an ion concentration of 10⁻⁶ mol/L is higher than the above range is designated as an electrode group A.

When the equilibrium potential of the aqueous solution as a dynamics inference system target varies within a region (c)-1, a group of electrodes having the equilibrium potential within the region 1 (in a region (c-3) in Fig. 8-2(c)) is designated as an electrode group B.

When the equilibrium potential of the aqueous solution as a dynamics inference system target varies within a region (c-1) in Fig. 8-2 (c), a group of electrodes having the equilibrium potential lower than those in the region (1) (in a region (c-4) in Fig. 8-2(c)) is designated as an electrode group C.

Here, at least one electrode may be selected from each of the electrode group A and the electrode group B and employed as the electrodes of the multi-electrode probe 11 (convolutional electrode probe).

In addition, at least one electrode may be selected from each of the electrode group B and the electrode group C and employed as the electrodes of the multi-electrode probe 11 (convolutional electrode probe). Furthermore, at least one electrode may be selected from each of the electrode group A, the electrode group B, and the electrode group C and employed as the electrodes of the multi-electrode probe 11 (convolutional electrode probe).

Examples of the combination of the above working electrodes will be described in detail below.

Fig. 9 illustrates a specific potential and pH relationship diagram. The equilibrium potential of the aqueous solution as a dynamics inference system target varies in a region (1) in Fig. 9. The electrodes in a region (2) (electrode group A) may be gold (Au) or platinum (Pt). The electrodes in a region (3) (electrode group B) may be argentum (Ag), copper (Cu), Nickel (Ni), cobalt (Co), or iron (Fe).

The electrodes in a region (4) (electrode group C) may be stainless steel (SUS), aluminum (Al), or titanium (Ti).

The following describes an example in a case where at least one electrode is selected from each of the electrode group A and the electrode group B.

Under the above condition (in the case where an electrode of the electrode group A and an electrode of the electrode group B are included), when three electrodes are selected, an Au electrode, a Pt electrode, and a Cu electrode may be included in the multi-electrode probe 11.

In this case, the Au electrode or the Pt electrode may function as the ground electrode 23, and the other electrodes may be the working electrodes.

Under the above condition (in the case where an electrode of the electrode group A and an electrode of the electrode group B are included), when four electrodes are selected, an Au electrode, a Pt electrode, a Cu electrode, and a Ni electrode may be included in the multi-electrode probe 11.

In this case, the Au electrode or the Pt electrode may function as the ground electrode 23, and the other electrodes may be the working electrodes.

The following describes an example in a case where at least one electrode is selected from each of the electrode group B and the electrode group C.

Under the above condition (in the case where an electrode of the electrode group B and an electrode of the electrode group C are included), when three electrodes are selected, a Cu electrode, a SUS electrode, and an Al electrode may be included in the multi-electrode probe 11.

In this case, the SUS or Al may function as the ground electrode 23, and the other electrodes may be the working electrodes.

Under the above condition (in the case where an electrode of the electrode group B and an electrode of the electrode group C are included), when three electrodes are selected, a Cu electrode, a Ni electrode, a SUS electrode, and an Al electrode may be included in the multi-electrode probe.

In this case, the SUS electrode or the Al electrode may function as the ground electrode 23, and the other electrodes may be the working electrodes.

The following describes an example in a case where at least one electrode is selected from each of the electrode group A, the electrode group B, and the electrode group C. Under the above condition (in the case where an electrode of the electrode group A, an electrode of the electrode group B, and an electrode of the electrode group C are included), when three electrodes are selected, a Pt electrode, a Cu electrode, and a SUS electrode may be included in the multi-electrode probe 11. In this case, the Pt electrode or the SUS electrode may be the ground electrode 23, and the other electrodes may be the working electrodes. Under the above condition (in the case where an electrode of the electrode group A, an electrode of the electrode group B, and an electrode of the electrode group C are included), when four electrodes are selected, a Pt electrode, a Cu electrode, a Ni electrode, and a SUS electrode may be included in the multi-electrode probe 11. In this case, the Pt electrode or the SUS electrode may be the ground electrode 23, and the other electrodes may be the working electrodes. Note that a casing (made of metal) of the multi-electrode probe 11 can be used as a ground electrode. In this case, each of the combinations described above may be selected as follows:
1) the number of electrodes is reduced by using the casing as the ground electrode;
2) while the ground electrode is used as it is, the casing is also used as a ground electrode, thereby reinforcing grounding performance; and/or
3) the casing is used as the ground electrode, and an electrode having a different electrode material is added.

### (Optimization Condition Inference Unit)

The system 1 for inference of dynamics of a system to be measured may have the optimization condition inference unit 28 for inferring the optimization condition of a dynamics inference system target (culture tank).

Inference processing of the optimization condition inference unit 28 may be executed using the learning estimation storage unit 41. The learning estimation storage unit 41 may have model data having parameters (what is called "weight") adjusted by performing machine learning on a lot of electrode information and dynamics observation data. For example, electrode data of the system 1 for inference of dynamics of a system to be measured and dynamics states of the dynamics inference system target (culture tank) 2 are used as teaching data to perform machine learning, such as deep learning, thereby creating the above model data. In this case, by referencing the above model data using the data of each electrode measured by the measurement system 38 as an input value, an optimal culture control condition of the dynamics inference system target (culture tank) can be obtained as an output value corresponding to the input value. The control system 39 of the system 1 for inference of dynamics of a system to be measured may have such a learned model in the learning estimation storage unit 41.

### <Information Processing Action (Overall Processing Flow)>

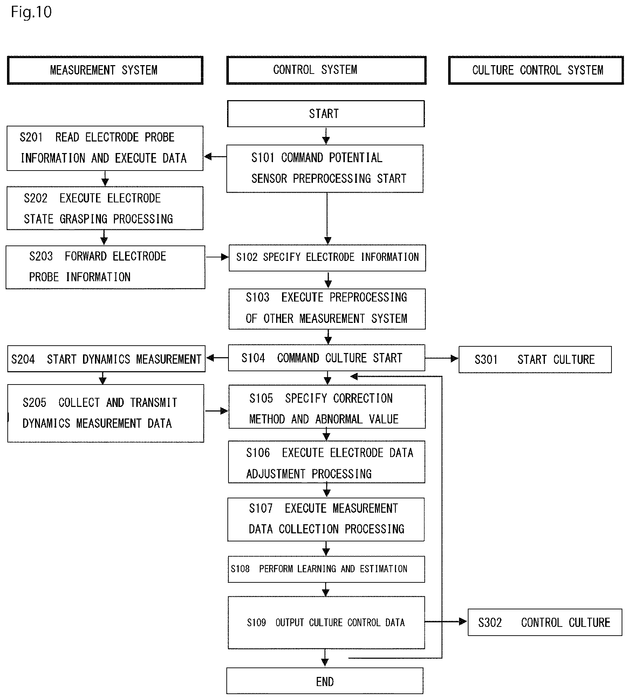

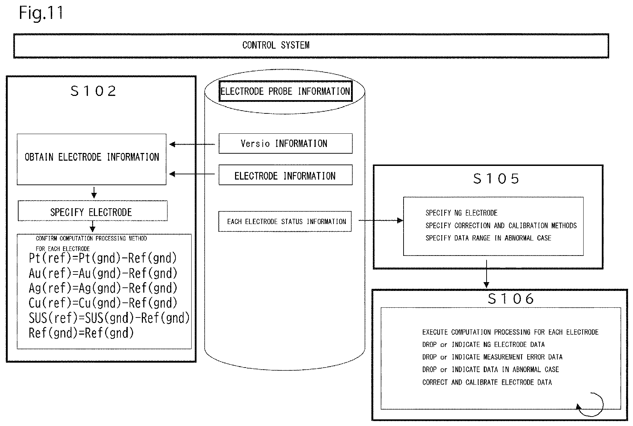

Subsequently, processing of an information-processing system according to an exemplary embodiment of the present invention will be described. Fig. 10 is a flowchart illustrating exemplary processing executed in the present invention. Fig. 11 a diagram illustrating a processing aspect in S102, S105, and S106 in Fig. 10 in more detail.

Note that the processing flow described below is an example of internal processing, and the internal processing that can be used for the system 1 of a system to be measured of the present invention is not limited to the example below. Processing from S101 to S109 below is processing executed by the control system 39, processing from S201 to S205 is processing executed by the measurement system 38, and processing from S301 to S302 is processing executed by the culture control system 40.

When a user operates to start preprocessing of a potential sensor via the operating unit of the control system, a command for potential sensor preprocessing start is transmitted from the control system 39 to the measurement system 38 via the communication unit (commanding potential sensor preprocessing start: S101).

When the measurement system 38 receives the above command, the control unit of the measurement system 38 reads the information of the multi-electrode probe 11 and electrodes (multi-electrode probe ID, version information, electrode ID, and electrode type) from the identification information recording unit 31 of the multi-electrode probe 11 and stores the information in the potential information and dynamics information storage unit 15 (reading and storing electrode probe information: S201). The values of the above multi-electrode probe ID and version information are registered in the multi-electrode probe table. The above multi-electrode probe ID, electrode IDs, and electrode types are registered in the electrode table.

Next, the control unit of the measurement system 38 executes state grasping processing of the above electrodes by the potential measuring and voltage applying unit 13 and the status confirmation unit 27 to acquire potential information and operating information of each electrode. The control unit of the measurement system 38 stores the above potential information and operating information (information on normal or not) of each electrode in the potential information and dynamics information storage unit 15 (electrode state grasping processing and data storing processing: S202).

Then, the measurement system 38 transmits the above information of the multi-electrode probe 11 and electrodes (information of the multi-electrode probe table and the electrode table) to the control system 39 (forwarding electrode probe information: S203). Afterwards, when the control system 39 receives the above information via the communication unit, the control unit of the control system 39 holds the above information in a temporary storage area or the like of the control system 39. Furthermore, at that time, based on the operation and the like of the control system 39 terminal by the user, the above information and electrode computation method for each electrode may be stored in the electrode table by the control unit (specifying electrode information: S102). Afterwards, the control unit of the control system performs a pH configuration and the like of the culture tank (preprocessing of other measurement instruments: S103).

Next, the control unit of the control system 39 transmits a message for culture start to the measurement system 38 and the culture control system 40 via the communication unit (commanding culture start: S104).

When the measurement system 38 receives the above message via the communication unit, the control unit of the measurement system starts measuring the electrode potentials and pH value of the dynamics inference system target 2 (starting dynamics measurement: S204).

The control unit of the measurement system 38 collects dynamics measurement data, such as the electrode potentials, stores the information in the dynamics information table of the potential information and dynamics storage unit 15, and transmits the information to the control system 39 via the communication unit (collecting and transmitting dynamics measurement data: S205).

Meanwhile, when the culture control system 40 receives the above message for culture start via the communication unit, the control unit of the culture control system 40 starts culturing the dynamics inference system target (culture tank) 2 (starting culture: S301).

After the above culture start command (S104), the control unit of the control system 39 references the electrode table and the potential information table to acquire the computation method, operating state, potential information, and electrode type for each electrode. Afterwards, based on the operation and the like of the control system 39 terminal by the user, the correction method and abnormal value data range may be stored in the electrode table by the control unit (specifying correction method and abnormal value data range: S105).

Afterwards, when the above information is handed over to the working electrode analyzing unit 35 from the control unit of the control system 39, based on the above computation method, the working electrode analyzing unit 35 performs the above computation for each electrode. When the above information is handed over to the electrode data adjustment unit 29 from the working electrode analyzing unit 35, based on the above information, the electrode data adjustment unit 29 creates and holds data having the electrode data corrected or excluded (exclusion or the like of abnormal electrode data) (electrode data adjustment processing: S106), and associates the data with the dynamics measurement data and store it in the learning estimation storage unit 41 (data collection processing: S107).

Afterwards, the control unit of the measurement system 38 performs learning and estimation of the dynamics by the optimization condition inference unit 28 based on the data acquired in S106 and S107 (learning and estimation: S108). The control unit of the measurement system 38 creates data for controlling the culture based on the above learning and estimation and transmits the data for controlling the culture to the culture control system via the communication unit (outputting culture control data: S109).

When the culture control system 40 receives the data for controlling the culture via the communication unit, the control unit of the culture control system 40 performs culture control based on the data (controlling culture: S302) .

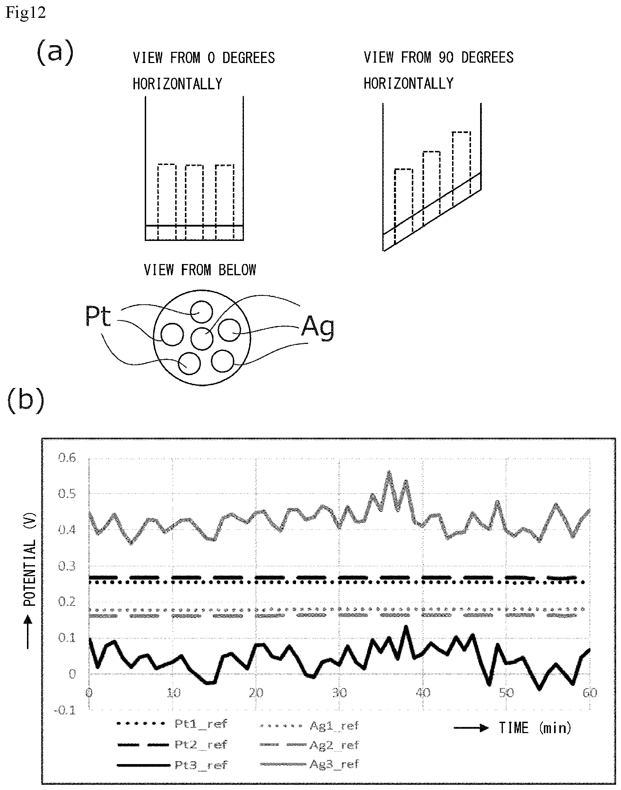

Fig. 12(a) is a conceptual diagram illustrating an example of an electrode probe having a plurality of electrodes made of an identical material. The example illustrated in Fig. 12(a) includes an argentum working electrode 1, a platinum working electrode 1, a platinum working electrode 2, an argentum working electrode 2, an argentum working electrode 3, and a platinum working electrode 3. The electrode probe is incorporated into a system as the electrode probe 11 illustrated in Fig. 5 to measure each electrode potential. Fig. 12 (b) is a graph substituting a drawing illustrating potentials measured using the system. The vertical axis of the graph is potential (V). The horizontal axis is time (min). The graph indicates the potentials of the working electrodes with reference (0 V) to a reference electrode. Relative potentials measured using the platinum working electrode 1 (black dotted line), the platinum working electrode 2 (black dashed line), the platinum working electrode 3 (black solid line), the argentum working electrode 1 (gray dotted line), the argentum working electrode 2 (gray dashed line), and the argentum working electrode 3 (gray solid line) to the reference electrode are indicated. In the example illustrated in Fig. 12(b), in the result, the two of the Pt and Ag electrodes exhibit similar values with a displaced amount within an allowable range. On the other hand, for both Pt and Ag, one electrode exhibits a significantly displaced value. In such a case, in edge processing, the significantly displaced one is removed as an abnormal case, and the average value of the remaining two electrodes is employed as the potential for Pt and Ag. In this way, an abnormal value can be removed. This processing may be automatically executed. For example, when a plurality of electrodes made of an identical material are included, potential differences used by the electrodes are stored in a storage unit. Then, the potential differences are read out and compared. As a result of the comparison, when any has a significant difference (for example, one exhibiting a threshold anomaly or different variation), the one is removed as an abnormal value. Then, the average of the potential differences derived from the electrodes other than the removed one as the abnormal value is obtained and output as the potential difference.

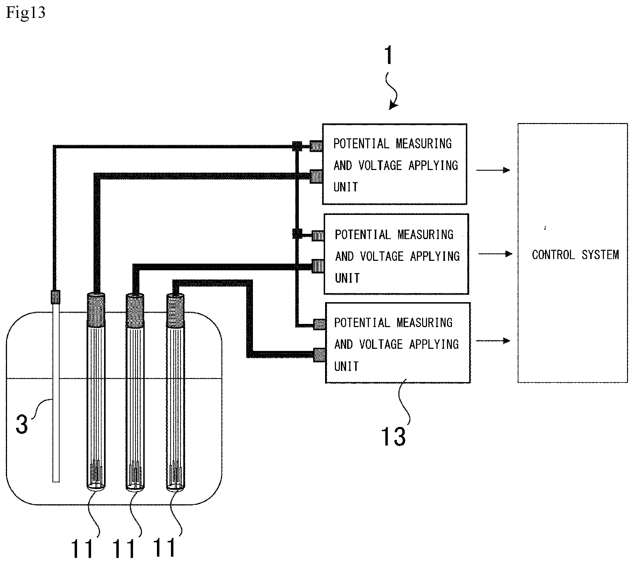

Fig. 13 is a conceptual diagram of a system including a plurality of multi-electrode probes. The system includes the reference electrode 3, a plurality of multi-electrode probes 11, and the potential measuring and voltage applying unit 13.

The potential measuring and voltage applying unit 13 measure the redox potential related to the reference electrode 3 and any one or at least two working electrodes included in the plurality of working electrodes 5, 7, and 9.

For each of the plurality of multi-electrode probes 11, the one previously described can be appropriately employed. For example, the multi-electrode probe 11 includes the plurality of working electrodes 5, 7, and 9 in a state of being insulated from one another and the distal end portion 17 that comes into contact with a measurement target. The plurality of working electrodes 5, 7, and 9 are exposed at least at the distal end portion 17. The plurality of working electrodes 5, 7, and 9 may be all made of an identical material. In addition, the plurality of working electrodes 5, 7, and 9 may be made of different materials. Furthermore, at least two or more of the plurality of working electrodes 5, 7, and 9 may be made of an identical material. Depending on the states of the electrodes and the state of a measurement system, measuring potentials exhibit abnormal values in some cases. By simultaneously measuring electrodes that are expected to originally exhibit an identical potential and comparing them, data in an abnormal case can be removed, and a highly accurate potential can be measured.

### INDUSTRIAL APPLICABILITY

The present invention, which relates to a system for inference of dynamics of a system to be measured, can be applicable in various technical fields such as experimental equipment industry, pharmaceutical industry, and biotechnology.

### REFERENCE SIGNS LIST

- 1: system for inference of dynamics
- 2: dynamics inference system target (culture tank)
- 3: reference electrode
- 5: first working electrode
- 7: second working electrode
- 9: third working electrode
- 11: multi-electrode probe
- 13: potential measuring and voltage applying unit
- 14: controller
- 15: potential information and dynamics information storage unit
- 17: distal end portion
- 21: connection unit
- 23: ground electrode
- 27: status confirmation unit
- 28: optimization condition inference unit
- 29: electrode data adjustment unit
- 31: identification information recording unit
- 33: identification information readout unit
- 35: working electrode analyzing unit
- 38: measurement system
- 39: control system
- 39a: control unit
- 39b: communication unit
- 39c: storage unit
- 39d: display unit
- 39e: operating unit
- 40: culture control system
- 41: learning estimation storage unit

## Claims

1. A system (1) for inference of dynamics of a system to be measured, comprising:
a reference electrode (3);
a multi-electrode probe (11) including a plurality of working electrodes (5, 7, 9) in a state of being insulated from one another, and
a potential measuring and voltage applying unit (13) that measure a redox potential related to the reference electrode (3) and any one or at least two working electrodes included in the plurality of working electrodes (5, 7, 9), wherein
the multi-electrode probe (11) has a distal end portion (17) in contact with a measurement target, and
the plurality of working electrodes (5, 7, 9) are exposed at least at the distal end portion (17).

2. The system (1) for inference of dynamics of a system to be measured according to claim 1, wherein
the distal end portion (17) has a flat shape.

3. The system (1) for inference of dynamics of a system to be measured according to claim 1, wherein
the distal end portion (17) has a shape inclined with respect to a longitudinal direction of the multi-electrode probe (11).

4. The system (1) for inference of dynamics of a system to be measured according to claim 1, further comprising
a connection unit (21) that removably connects the multi-electrode probe (11), wherein
the potential measuring and voltage applying unit (13) are connected to the multi-electrode probe (11) via the connection unit (21).

5. The system (1) for inference of dynamics of a system to be measured according to claim 1, wherein
the multi-electrode probe (11) further includes a ground electrode (23) further insulated from the plurality of working electrodes (5, 7, 9).

6. The system (1) for inference of dynamics of a system to be measured according to claim 1, wherein
each of the plurality of working electrodes (5, 7, 9) has a diameter of 0.5 mm or more and 3 cm or less.

7. The system (1) for inference of dynamics of a system to be measured according to claim 1, wherein
the respective plurality of working electrodes (5, 7, 9) have different ionization tendencies.

8. The system (1) for inference of dynamics of a system to be measured according to claim 1, further comprising
a status confirmation unit (27) that performs status confirmation of the system (1) for inference of dynamics of a system to be measured based on the redox potential measured by the potential measuring and voltage applying unit (13).

9. The system (1) for inference of dynamics of a system to be measured according to claim 8, wherein
the potential measuring and voltage applying unit (13) applies a plurality of types of voltages to at least one of the plurality of working electrodes (5, 7, 9),
and, based on the redox potential when the potential measuring and voltage applying unit (13) apply the plurality of types of voltages to at least one of the plurality of working electrodes (5, 7, 9),the status confirmation unit (27) performs status confirmation of the system (1) for inference of dynamics of a system to be measured.

10. The system (1) for inference of dynamics of a system to be measured according to claim 1, wherein
the multi-electrode probe (11) further includes an identification information recording unit (31) that stores identification information of the multi-electrode probe (11),
the system (1) for inference of dynamics of a system to be measured further includes:
an identification information readout unit (33) that reads out the identification information of the multi-electrode probe (11) from the identification information recording unit (31); and
a working electrode analyzing unit (35) that analyzes the plurality of working electrodes (5, 7, 9) included in the multi-electrode probe (11) based on the identification information of the multi-electrode probe (11) read out by the identification information readout unit (33) .

11. The system (1) for inference of dynamics of a system to be measured according to claim 1, wherein
the plurality of working electrodes (5, 7, 9) includes two or more working electrodes having an identical cross-sectional shape and made of identical material.

12. A system (1) for inference of dynamics of a system to be measured, comprising:
a reference electrode (3);
a plurality of multi-electrode probes (11); and
a potential measuring and voltage applying unit (13) that measure a redox potential related to the reference electrode (3) and any one or at least two working electrodes included in the plurality of working electrodes (5, 7, 9), wherein
each of the plurality of multi-electrode probes (11) includes
a plurality of working electrodes (5, 7, 9) in a state of being insulated from one another and a distal end portion (17) in contact with a measurement target, and
the plurality of working electrodes (5, 7, 9) are exposed at least at the distal end portion (17).
